(19) Europäisches Patentamt / European Patent Office / Office européen des brevets

(11) **EP 3 609 393 B1**

## (12) EUROPEAN PATENT SPECIFICATION

(45) Date of publication and mention
of the grant of the patent:
**28.05.2025   Bulletin 2025/22**

(21) Application number: **18784766.0**

(22) Date of filing: **03.04.2018**

(51) International Patent Classification (IPC):
**A61B 5/021** (2006.01)       **A61B 5/00** (2006.01)

(52) Cooperative Patent Classification (CPC):
**A61B 5/02116;** A61B 5/726; A61B 5/7264;
A61B 5/7282; A61B 2560/0223

(86) International application number:
**PCT/IB2018/052297**

(87) International publication number:
**WO 2018/189622 (18.10.2018 Gazette 2018/42)**

(54) **NON-INVASIVE BLOOD PRESSURE MEASUREMENT**

NICHTINVASIVE BLUTDRUCKMESSUNG

MESURE NON EFFRACTIVE DE LA TENSION ARTÉRIELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.04.2017   US 201762485128 P**

(43) Date of publication of application:
**19.02.2020   Bulletin 2020/08**

(73) Proprietor: **Atcor Medical Pty Ltd
Sydney NSW 2000 (AU)**

(72) Inventor: **QASEM, Ahmad
Guildford, New South Wales 2161 (AU)**

(74) Representative: **Cleveland Scott York
5 Norwich Street
London EC4A 1DR (GB)**

(56) References cited:
WO-A1-2015/174499       US-A1- 2011 275 944
US-A1- 2012 277 602       US-A1- 2016 220 194
US-A1- 2016 242 700       US-A1- 2017 112 395
US-A1- 2017 181 649       US-A1- 2018 116 600

• SONE SHUSAKU ET AL:
"Photoplethysmography and ultrasonic-measurement-integrated simulation to clarify the relation between two-dimensional unsteady blood flow field and forward and backward waves in a carotid artery", MEDICAL AND BIOLOGICAL ENGINEERING AND COMPUTING, SPRINGER, HEILDELBERG, DE, vol. 55, no. 5, 27 July 2016 (2016-07-27), pages 719 - 731, XP036237526, ISSN: 0140-0118, [retrieved on 20160727], DOI: 10.1007/S11517-016-1543-4

**Description**

FIELD OF THE INVENTION

The invention pertains to measuring systolic and diastolic blood pressure non-invasively, without using a brachial cuff operating in oscillometric mode. The invention is directed to calibrating a non-invasive arterial pulse waveform based on the shape of a scaled version of the waveform so that its maximum and minimum values accurately estimate the patient's systolic (SP) and diastolic blood pressure (DP). Also disclosed herein is a clinical classification for which the patient's SP and DP are expected to qualify, such as optimal, normal, high normal, and grade of hypertension.

BACKGROUND OF THE INVENTION

Arterial blood pressure is a clinically important indicator of the status of the cardiovascular system, reflective of arterial and cardiac load and an early independent predictive marker of cardiovascular events and diseases. However, to measure the inter-arterial blood pressure accurately requires an invasive procedure to insert a catheter with a pressure sensor inside the artery. As a result, non-invasive methods were created to estimate pressure at the peripheral brachial artery.

One of the earliest non-invasive methods to estimate pressure in the brachial artery is the auscultatory method which requires inflating a cuff wrapped around the patient's upper arm and brachial artery until the brachial artery occludes (i.e., no blood flow). Then, the cuff is gradually deflated and blood starts flowing with "thumping" sounds that can be detected through a stethoscope. The first "thumping" sound should occur when the cuff pressure equals the patient's systolic pressure (maximum pressure during cardiac ejection) and the last "thumping" sound should occur when the cuff pressure equals the patient's diastolic pressure (minimum pressure during cardiac filling).

For decades, the auscultatory method was used for clinical hypertension diagnosis and had become the standard for non-invasive blood pressure measurement. However, the accuracy of the measured pressure value was dependent on the operator's acute detection of the heart sound and also dependent on the rate that the operator deflates the cuff. Because the accuracy of the auscultatory method is operator dependent, an automated method was established based on detecting oscillatory pulsations measured by the brachial cuff during cuff inflation or deflation. The height of the pulse oscillation increases when the cuff pressure decreases from systolic pressure to below systolic pressure and the height of the oscillation decreases when the cuff pressure decreases from above diastolic pressure to diastolic pressure and below. Based on this concept, current "oscillometric" devices apply different algorithms to detect oscillation heights related to systolic and diastolic pressure.

Oscillometric cuff devices are often called non-invasive blood pressure devices or NIBP devices in the art. To be accepted for clinical use, an NIBP device has to show equivalence to the standard auscultatory method based on the American National Standard for Non-Invasive Automated Blood Pressure Devices, ANSI/AAMI/ISO 81060-2:2009, "Non-invasive sphygmomanometers- Part 2: Clinical validation of automated measurement type," Section 5.2.4.1.2 Part a-Criterion 1, page 20 (which states that the mean error for determination of all subjects in the test "shall not be greater than 5.0 mmHg with a standard deviation no greater than 8 mmHg.") Accordingly, any oscillometric cuff device can pass the validation requirements if the average difference with the auscultatory method for systolic and diastolic pressure is not more than 5 mmHg and the standard deviation is not more than 8 mmHg. This means that approved oscillometric devices can register a difference with the standard auscultatory method reaching above 20 mmHg for some data points.

Oscillometric automated blood pressure devices have been standard in clinical practice for many years, and have also been used in medical research to assess cardiovascular risk. Even though non-invasive blood pressure (NIBP) measurement identifies a percentage of the general population at risk of cardiovascular diseases, a large group is not identified by NIBP measurement to be at risk even though they may be at risk. The main reason is that measured blood pressure varies among different NIBP devices due to the different devices having different propriety algorithms for detecting systolic and diastolic pressure. Furthermore, when compared to invasive pressure values, NIBP devices have been shown to underestimate systolic pressure and overestimate diastolic pressure, see, Sharman et al., "Validation of non-invasive central blood pressure devices: Artery Society task force consensus statement on protocol standardization", European Heart Journal (2017) 0, 1-10; Cloud et al., "Estimation of central aortic pressure by SphygmoCor® requires intra-arterial peripheral", Clinical Science (2003) 105, 219-225; Shoji et al., "Invasive validation of a novel brachial cuff-based oscillometric device (SphygmoCorXCEL) for measuring central blood pressure", Journal of Hypertension 2016, 34. Accordingly, since measuring brachial pressure invasively is the gold standard, non-invasive measurements that closer estimate the invasive pressure and overcome the errors inherent in cuff NIBP devices would be a significant improvement in the field of blood pressure measurement and its clinical importance.

First, as mentioned, with the maximum acceptable error standard deviation (see ANSI/AAMI/ISO 81060-2:2009, "Non-invasive sphygmomanometers- Part 2: Clinical validation of automated measurement type", Section 5.2.4.1.2 Part a- Criterion 1, page 20) being 8 mmHg for a statistically approved NIBP cuff device, the device may have an error of 10 mmHg or above on about 20-30% of the general population. This relatively high margin of error means that some subjects with cardiovascular risk are classified as healthy and some are classified as healthy when they should in fact be classified as at risk.

Second, invasive pressure data has shown that the difference between cuff NIBP and invasive brachial artery SP and DP typically has either a high average error or high error standard deviation that would exceed 15 mmHg on a large percentage of the study population (see, Cloud et al. and Shoji et al. referenced above). These errors reduce NIBP reliability significantly in clinical practice.

Third, different cuff NIBP devices use different algorithms to detect SP and DP from cuff oscillatory pulses, which results in variations between the NIBP devices' measurements adding to cuff NIBP unreliability.

Fourth, given that blood pressure and heart rate continuously adjust based on the body's demand due to metabolism, blood pressure and heart rate are not constant and can change from beat to beat. The continuous monitoring of beat-to-beat blood pressure, like heart rate with ECG devices, would provide a useful blood pressure variability assessment tool, such as the ability to immediately detect sudden changes in blood pressure that allows prompt medical staff response. Like heart rate monitors providing an ECG, devices monitoring beat-to-beat blood pressure will be clinically valuable. Yet, the cuff NIBP measurements, which take about 30 seconds to 2 minutes to measure SP and DP, do not measure blood pressure continuously beat by beat. Furthermore, blood pressure may change during the cuff NIBP duration of blood pressure measurements producing inaccurate blood pressure values.

Fifth, the oscillometric cuff NIBP devices require the cuff to be inflated above SP occluding the brachial artery and seizing blood flow for few moments which may cause patient's discomfort. Even though the cuff NIBP devices are low risk devices, such inconvenience may also affect blood pressure which the device is trying to measure.

As a result, attempts have been made to estimate SP and DP without using cuff NIBP in order to provide continuous blood pressure measurements without the inconvenience of a cuff obstructing and disturbing brachial arterial blood flow. One of the most common methods (Masè et al., Journal of Electrocardiology 2011-44 pp 201-207; Chen et al., Annals of Biomedical Engineering 2012, Vol. 40, No. 4, pp. 871-882; Zheng et al., J Med Syst 2016, 40:195; Fuke et al., Zheng et al., and Solà et al., 35th Annual International Conference of the IEEE EMBS 2013 July) is detecting SP and DP by measuring the pulse wave velocity (PWV) or pulse transit time (PTT) between two simultaneously measured arterial pulses or between a simultaneously measured ECG signal and an arterial pulse. These methods are based on the fact that pulse wave velocity, which is calculated from PTT, is related to pressure. Accordingly, by measuring PTT, blood pressure can be estimated or detected. However, the method requires calibration with a cuff NIBP device for the first PTT measurement on any setting, like a different patient or different patient's posture, because the relationship between PTT and blood pressure is related to change. After calibration, the initial PTT is associated with SP and DP values and any changes in PTT afterward relate to changes in blood pressure. The method still requires a cuff NIBP every time it is used in different settings, like for a different patient or different patient's posture, which means the method is not totally cuff-less. Another issue with the method is that it requires simultaneous recordings of two signals at different positions, which adds complication in the hardware design to assure accuracy of the recordings let alone the inconvenience of having sensors at two arterial locations.

Another method was proposed by Baruch (U.S. 8,100,835 B2) to estimate SP and DP from one arterial pulse recording. The method consisted of decomposing and then identifying three (3) peaks from a recorded radial pulse. The method relates the time between the peaks with SP and DP. Implementing such a method faces the same issue faced with the PTT methods, namely, the need for calibration or individualizing the method. The method by Baruch identified that the linear relationship between the time between the peaks in the arterial pulse recording and SP and DP is different between different subjects in the population. The solution according to Baruch is to have different linear relationships based on gender, height, disease status, fitness or/and any other parameters in the patient's profile. Individualizing the method this way is impractical and renders the detection of SP and DP from a pulse redundant because the patient's profile will be the main determents of SP and DP.

Another method by Lading et al. (U.S. Patent Application US 2015/0327786 A1) estimates pulse pressure PP, which is equal to SP-DP, and mean pressure from changes in the cross sectional area distension related to the pulse in a peripheral artery (e.g., brachial, radial or finger). The method first requires recording of two measurements of the peripheral arterial distension pulse at different hydrostatic pressures (hand down and hand raised at the heart level) to determine the relationship between the recorded changes in arterial area distension with pressure in relation to a known hydrostatic pressure. This maneuver is a form of calibration. The method also fits an exponential decay curve on the diastolic portion of the arterial distension pulse to estimate initial values of PP and distension to pressure conversion coefficients.

The Lading et al. method suffers from the following issues that impact its practical general implementations. First, before any measurement, multiple measurements of hydrostatic pressure and the level of arterial distension related to the pulse need to be performed. Second, in order for the method to be accurate, measurement requires multiple sensors, namely, a sensor to record the arterial distension pulse and an elevation sensor to record hydrostatic pressure. The method also suffers from other issues affecting its accuracy. The method requires a measurement of the amount of arterial distension related to the pulse, however, the method fails to address that many sensors signals do not measure direct arterial distension pulse but a combination of flow, pressure and volume which are all variables affecting the assumed linear relationship between arterial distension and pressure and consequently the accuracy of the estimated SP and DP.

US2012277602 (Tichauer Larry Martin), US2016220194 (Samsung Electronics), and SONE SHUSAKU ET AL, "Photoplethysmography and ultrasonic-measurement-integrated simulation to clarify the relation between two-dimensional unsteady blood flow field and forward and backward waves in a carotid artery", Medical And Biological Engineering And Computing, Springer, Heildelberg, DE, vol. 55, no. 5, doi:10.1007/S11517-016-1543-4, ISSN 0140-0118, (20160727), pages 719 - 731, (20160727), disclose methods involving non-invasively sensing and recording an un-calibrated pulse waveform with sufficient fidelity to preserve the cardiovascular features of the waveform. The current invention distinguishes from the prior art as it requires a single high-fidelity, non-invasive, un-calibrated peripheral or central arterial pressure or pressure related pulse waveform to estimate SP and DP or hypertension (DP/SP) class. The invention can calculate SP and DP, or determine a hypertension (DP/SP) class, from the non-invasive waveform measurement with no requirement for maneuver or cuff NIPB calibration.

## SUMMARY OF INVENTION

In one aspect, the invention pertains to a method of non-invasively measuring a patient's systolic and diastolic blood pressure, which avoids the disadvantages facing present day brachial cuff NIBP devices operating in oscillometric mode, in accordance with the appended claims.

To implement this aspect of the invention, an un-calibrated pulse waveform with sufficient fidelity to preserve cardiovascular features of the waveform is non-invasively sensed and recorded. The pulse waveform can be sensed at a peripheral location or a central location depending on the embodiment of the invention. The term pulse waveform is used herein to mean both pressure pulse waveforms and pressure-related pulse waveforms such as a volumetric displacement waveform from a brachial cuff. The pulse waveform can be measured using a non-invasive sensor such as a tonometer, plythsmograph, bio-impedance sensor, photodiode sensor, RF sensor or sonar Doppler sensor on a peripheral artery like a radial artery, a brachial artery, finger or a central artery such as a carotid artery. In this regard, the invention provides the capability of a cuffless solution to accurately measure SP and DP. On the other hand, the invention can also be used with a cuff to record a brachial volumetric displacement waveform.

The recorded, un-calibrated pulse waveform is then scaled such that the amplitude of the scaled waveform is a set to a fixed value. For example, the minimum of the waveform can be set to Mn=0 and the peak of the waveform can be set to Mx=100. An average waveform taken over several data cycles is desirably used as the un-calibrated waveform prior to scaling.

The scaled waveform is then calibrated based on one or more cardiovascular features in the scaled waveform. This calibration is implemented by an algorithm that accurately correlates the non-invasively recorded, un-calibrated and scaled waveform to collected data based on the cardiovascular features in the scaled waveform. In some embodiments of the invention, the algorithm correlates the waveform to invasively collected data, and in other embodiments of the invention the algorithm correlates the waveform to non- invasively collected data (e.g. collected with a conventional brachial blood pressure cuff device). Linear models like auto-regressive models or/and non-linear models like nonlinear system identification and machine learning methods like decision tree, or support vector machine are used to develop the algorithm capable of implementing the invention. The calibration is able to shift and scale the amplitude of the waveform so that the minimum of the calibrated waveform accurately estimates DP and the peak of the calibrated waveform accurately estimates SP as if DP and SP were measured directly, either invasively or non-invasively (e.g. conventional brachial blood pressure cuff device) as the case may be. Accordingly, the patient's SP is estimated as the maximum value of the calibrated waveform and the patient's DP is estimated as the minimum value of the calibrated waveform.

Also disclosed herein is a method of providing a patient's blood pressure status. More specifically, the method identifies the patient's hypertension (SP/DP) classification (e.g. Optimal, Normal, High Normal, Grade I HT, Grade II HT), again with a technique that avoids the disadvantages facing present day brachial cuff NIBP devices operating in oscillometric mode. To implement this aspect of the invention, an un-calibrated pulse waveform with sufficient fidelity to preserve cardiovascular features of the waveform is non-invasively sensed and recorded as described above. Again, the pulse waveform can be sensed at a peripheral location or a central location depending on the embodiment of the invention. The pulse waveform can be measured using a non-invasive sensor such as a tonometer, plythsmograph, bio-impedance sensor, photodiode sensor, RF sensor or sonar Doppler sensor on a peripheral artery like a radial artery, a brachial artery, a finger or a central artery like a carotid artery. This aspect of the invention similarly provides the capability of a cuffless solution, although a cuff can be used to record a brachial volumetric displacement waveform when implementing this aspect of the invention.

Again the recorded, un-calibrated pulse waveform is then scaled such that the amplitude of the scaled waveform is a set to a fixed value. For example, the minimum of the waveform can be set to Mn=0 and the peak of the waveform can be set to Mx=100. An average waveform taken over several data cycles is desirably used as the un-calibrated waveform prior to scaling.

At this point in the process, the method according to this aspect of the invention is different from the method according to the first aspect of the invention. When implementing this aspect of the invention, parameter values are determined for one or more cardiovascular features of the scaled waveform. A classification algorithm correlates the parameter values determined for one or more cardiovascular features of the scaled waveform to multiple hypertension classifications (e.g. Optimal, Normal, High Normal, Grade I HT, Grade II HT). Linear models like auto-regressive models or/and non-linear models like nonlinear system identification and machine learning methods like decision tree, or support vector machine are used to develop the classification algorithm. Accordingly, one of the multiple hypertension classifications is selected based on the parameter values of the one or more cardiovascular features determined from the scaled waveform using the classification algorithm, and the selected hypertension classification is displayed for the viewing.

The invention can be implemented using a digital signal processor and a computer with a monitor. It can also be implemented, in whole or in part, as wearable device that can continuously and accurately measure either SP and DP or a hypertension classification.

BRIEF DESCRIPTION OF THE DRAWINGS

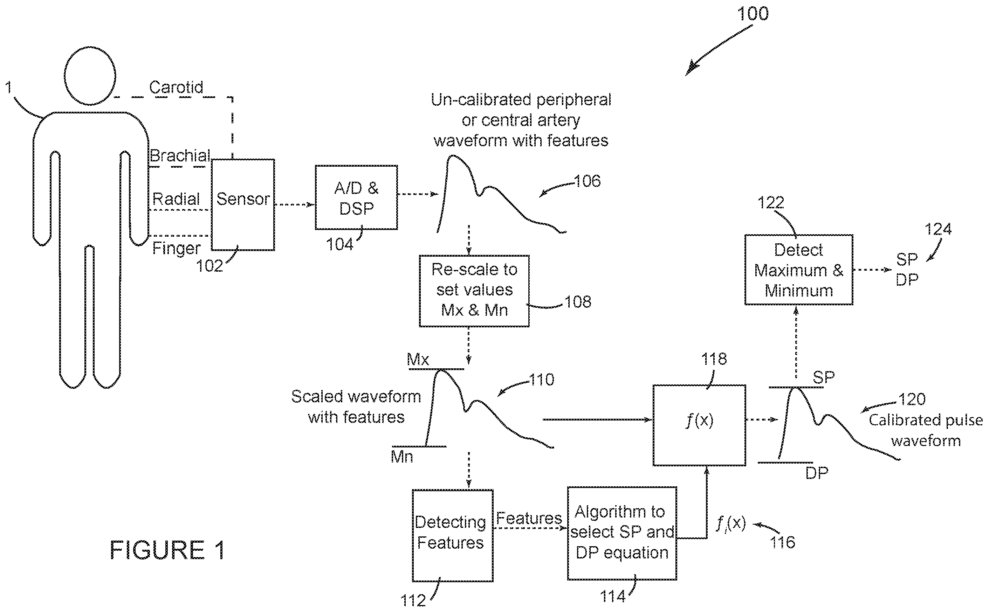

Figure 1 is the schematic drawing of an implementation of one embodiment of the invention which in general consists of recording a non-invasive peripheral (e.g., brachial, radial or finger) or central (e.g., carotid), arterial pulse waveform, rescaling the waveform with set values, detecting cardiovascular related features from the scaled waveform, applying an algorithm to the values of the cardiovascular features to select which equation should be used to calculate a calibrated waveform from the scaled waveform, applying the selected equation to the scaled waveform, and a detecting the maximum and minimum of the outputted calibrated waveform as the SP and DP respectively.

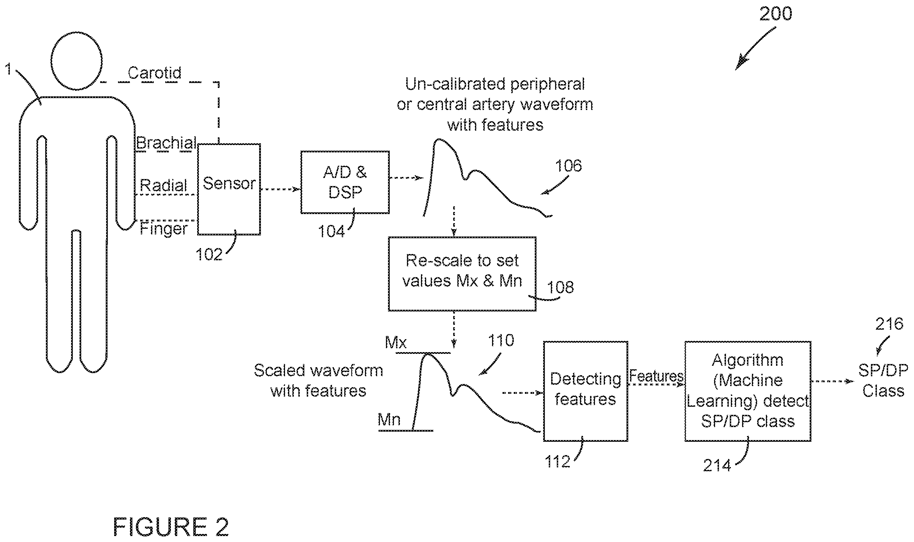

Figure 2 is the schematic drawing of an implementation of another embodiment of the invention which in general consists of recording a non-invasive peripheral (e.g., brachial, radial or finger) or central (e.g., carotid) artery pulse waveform, rescaling the waveform with set values, detecting cardiovascular related features from the scaled waveform, and applying an algorithm on the values of the cardiovascular features that determines a clinical classification for which the patient's SP and DP are expected to qualify, such as optimal, normal, high normal, and grade of hypertension.

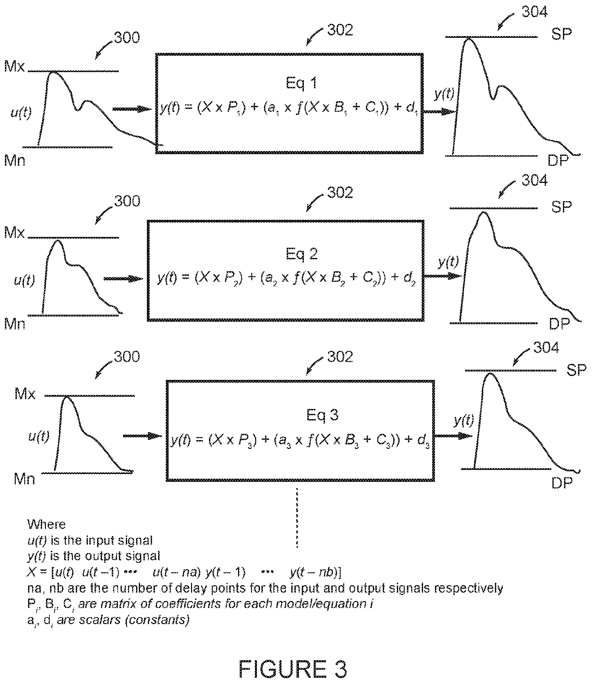

Figure 3 shows the form of calibration equations determined for scaled peripheral or central arterial pulse waveforms with set values Mx and Mn. The calibration equations produce a calibrated waveform where the maximum and minimum correspond accurately to directly measured SP and DP respectively.

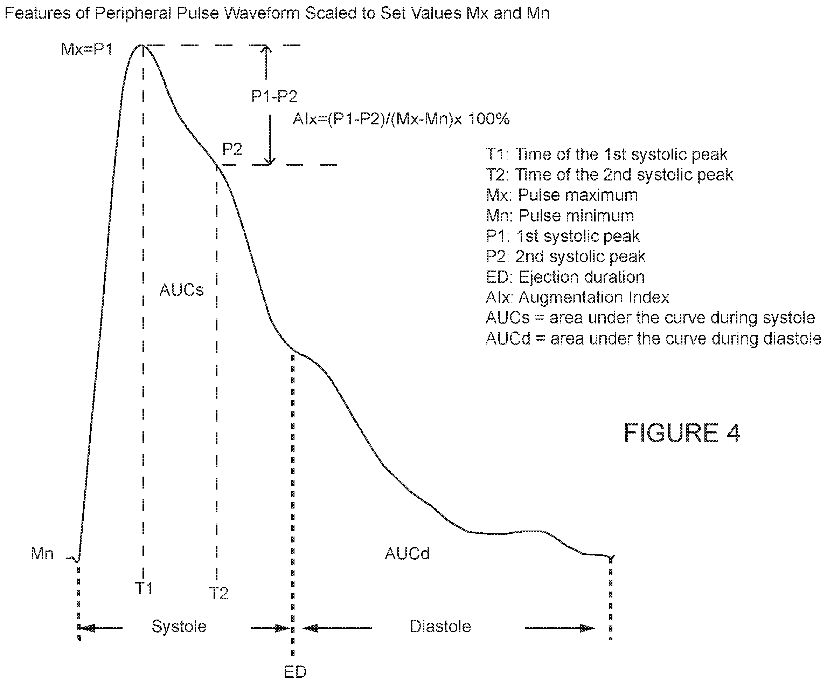

Figure 4 illustrates some cardiovascular related features of a non-invasive peripheral arterial pulse waveform (some of them having been detailed in U.S. Patent No 5,265,011), which are used when implementing the embodiments of the invention illustrated in Figures 1 and 2 using a non-invasive sensor to measure a pulse waveform in a peripheral artery.

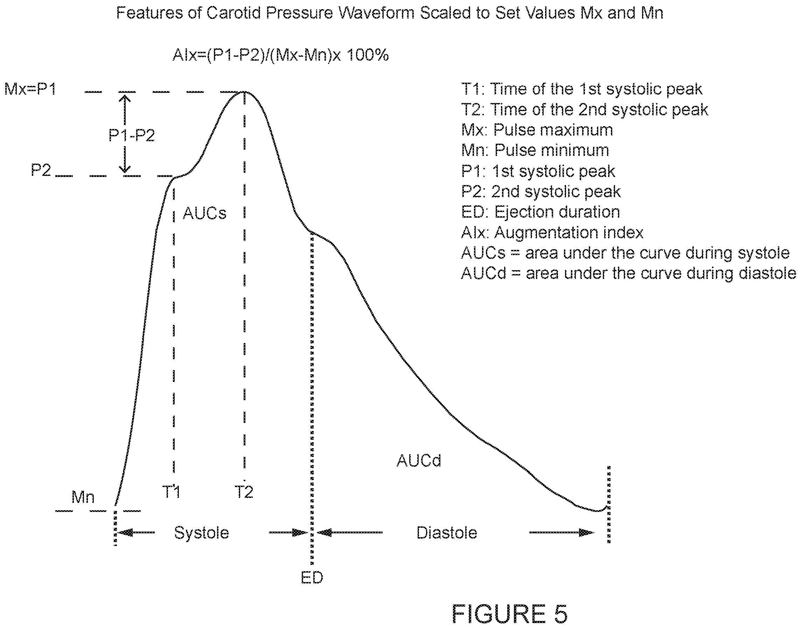

Figure 5 illustrates some cardiovascular related features of a non-invasive central artery pulse waveform (some of them having been detailed in U.S. Patent No 5,265,011). The cardiovascular related features are used when implementing the embodiments of the invention illustrated in Figures 1 and 2 using a non-invasive sensor to measure a pulse waveform in the carotid artery.

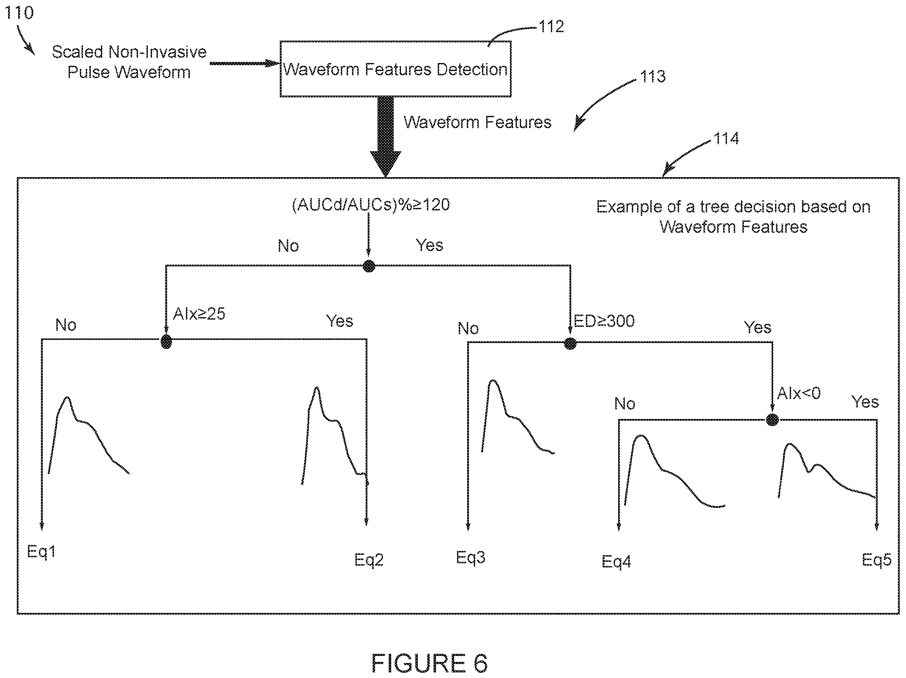

Figure 6 shows an example of a decision tree that selects the appropriate calibration equation for applying to the scaled waveform based on the values of the cardiovascular features of the scaled waveform.

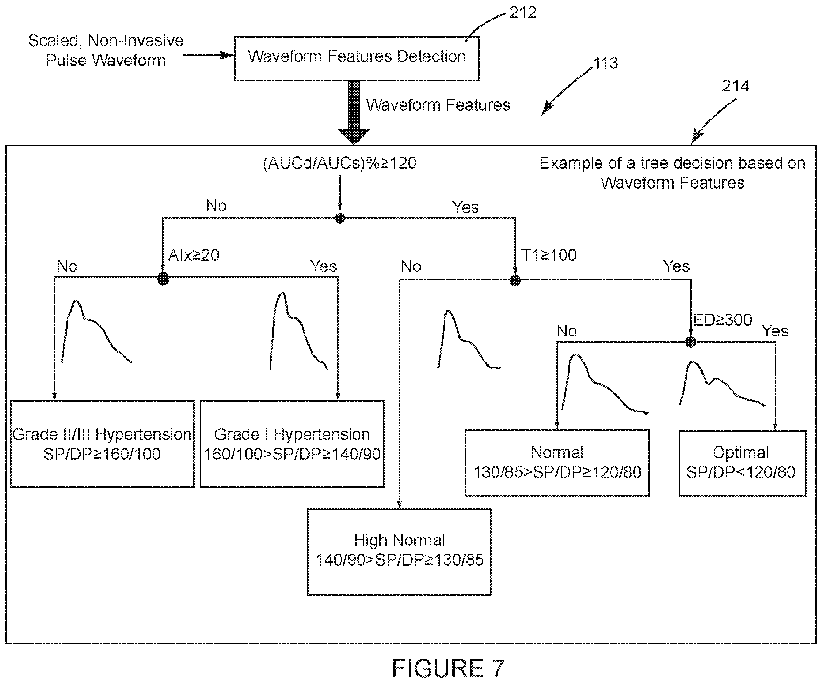

Figure 7 shows an example of a decision tree that selects an appropriate clinical classification for which the patient's SP and DP are expected to qualify, such as optimal, normal, high normal, and grade of hypertension, based on the values of the cardiovascular features of the scaled waveform.

DETAILED DESCRIPTION

Figure 1 shows a system 100 configured in accordance with a first embodiment of the invention. This embodiment requires a sensor 102 to non-invasively record an arterial pulse waveform. The term pulse waveform, as mentioned above, includes pressure pulse waveforms as well as other pulse waveforms such as volumetric displacement waveforms. Figure 1 indicates that the non-invasive pulse waveform can be measured at a central location such as the carotid artery or a peripheral location such as the brachial or radial artery or in the finger. Various non-invasive sensors 102 can be used such as a tonometer, plethysmograph, bio-impedance, Doppler sensor or brachial cuff device to record non-invasive pressure or pressure related arterial pulse waveform from a peripheral artery (like finger, radial or brachial artery) or a central artery (like carotid artery).

One of the objects of the invention is to avoid measuring SP and DP with a NIBP cuff device operating in oscillometric mode; however, a cuff device can be used in accordance with the invention to capture a high-fidelity, brachial volumetric displacement waveform, as described in the Qasem U.S. Patent No. 9,314,170, initially published as US 2011/0275944 A1.

It is contemplated that the sensor 102 could be a wearable sensor such as a tonometer, plythsmograph, bio-impedance, photodiode sensor, RF sensor or Doppler sensor, that records the non-invasive pressure or pressure related arterial pulse waveform from a peripheral artery or a central artery.

Through the A/D & DSP unit 104, the recorded analogue signal is converted into a digital signal and digitally processed by applying suitable high pass, low pass, band pass filters or combination of these filters in order to produce a high-fidelity, un-calibrated waveform 106 with cardiovascular related features preserved.

In another embodiment, the sensor 102 records continuous pulses for a specified amount of time (e.g., 5 or 10 seconds) and the DSP units (2) converts the string of pulses into digital data, and filters the data high pass, low pass, band pass filters or combination of these filters, and (3) then averages all the pulses to obtain a single average pulse waveform with cardiovascular related features preserved.

In one alternative, the sensor 102 can be a NIBP cuff device that measures non-invasive systolic and diastolic pressures (NISP and NIDP respectively) and records a raw oscillometric cuff waveform while the cuff is inflated to a constant pressure (below NIDP, between NIDP and NISP or above NISP). The raw signal from the NIBP cuff unit is sent to the digital signal processor 104, which filters the signal to ensure that the cardiovascular waveform features are preserved and converts the waveform to digital data for processing. As discussed above, the raw cuff waveform is processed through a high pass filter and low pass filter or a band pass filter to produce an un-calibrated brachial cuff waveform with cardiovascular related features preserved. This waveform is a brachial cuff volumetric displacement waveform, which contains and preserves the cardiovascular features present in the patient's brachial pressure waveform. The pressure of the inflated cuff will affect the shape of the recorded waveform; and therefore it is important that the cuff be inflated to a range consistent with the inflation of the cuff for the data collected to determine the calibration equations discussed below. In particular, the shape changes significantly depending on whether the cuff is inflated below the patient's DP, between DP and SP or above SP. For example, if the calibration equations are determined based on data collected with the cuff inflated below diastolic pressure for the test population, then the raw brachial (volumetric displacement) waveform should be collected with the cuff inflated below the patient's diastolic. It is preferred that the inflated cuff pressure have a 10% difference or more compared the patient's DP in order to avoid borderline effects. The same considerations apply with respect to both DP and SP in the case that the recalibration equations are determined based on data collected with the cuff inflated between DP and SP for the test population, or with respect to SP in the case that the calibration equations are determined based on data collected with the cuff inflated above SP for the test population. It is possible that a non-invasive waveform 106 captured using a pressure sensor like a tonometer may not need much filtering. On the other hand, if a brachial cuff device is used to capture the raw un-calibrated waveform, substantial filtering may be required. While the filtering of the raw cuff waveform is dependent on the particular cuff device, the cuff pressure relative to NISP or NIDP and NIBP unit used, the filtering in an exemplary embodiment uses a low pass filter with cutoff frequency between 30 to 40 Hz, and high pass filter with pass frequency between 0.7 to 1 Hz has been found suitable to capture a raw waveform in which the cardiovascular features, including the foot, first systolic peak, second systolic peak and incisura, are preserved in the data. The purpose of the low pass filter is to preserve volume, pressure or flow signal frequencies that are related to physiological function and eliminate noises related to environmental inferences such as power sources noise. The choice of the low pass cutoff frequency is based on the fact that all physiological features in pressure, volume or flow waveforms are within 25 Hz of the signal spectrum (See Figure 26.21 in W. Nichols and M. O'Rourke, "McDonald's Blood Flow in Arteries: Theoretical, Experimental and Clinical Principles", 5th Edition). The purpose of the high pass filter is to eliminate low frequencies related to artifacts noise as a result of arm movements, breathing effect or the tube and cuff compliance in reaction to pressure. These low frequency artifacts, which cause signal baseline drift and can dampen signal shape, are usually below 1 Hz, hence the high pass filter pass frequency. Both filters, which can be implemented as a Chebyshev type filters with pass band ripple or stop band ripple of -3dB, can be combined into one band pass filter where it pass all frequencies between 0.7 to 40 Hz.

The operations after block 104 in Figure 1 are also preferably implemented in a digital signal processor 104, or other computing device. However, the electronic filters discussed in connection with acquiring the raw waveform can be analog or digital, or a combination of both.

Block 108 represents software that rescales the un-calibrated peripheral (or central) waveform 106 such that its maximum and minimum are set equal to pre-set scaling values Mx and Mn , which can be any number such as Mx=100 and Mn=0. The result is a scaled waveform 110 in which the cardiovascular features are preserved.

Block 112 depicts the scaled pulse waveform 110 being input for an algorithm to detect parameter values for identified cardiovascular features of the scaled waveform 110. Some of these cardiovascular features have been described in U.S. Patent No 5,265,011 and are described below in connection with Figure 4 (scaled peripheral waveform) and Figure 5 (scaled central waveform). The algorithm 112 can detect cardiovascular features using the derivative method as described in U.S. Patent No. 5,265,011, the wavelet method, or any other suitable method. The detected features from block 112 are the input for an algorithm 114 that selects one of several calibration equations *fi(x)* 116 to calibrate the scaled waveform resulting in a calibrated waveform 120 (peripheral or central depending whether the sensor 102 and the algorithm 114 used to detect the cardiovascular features are specific for a peripheral waveform or a central waveform). The algorithm 114 shifts and/or scales the scaled waveform 110, so that its minimum value corresponds to the patient's arterial DP and its maximum corresponds to the patient's arterial SP. The selection algorithm 114 and the calibration equations $f_i(x)$ 116, as illustrated in Figures 6 and 3, are described in more detail below. Block 118 in Figure 1 indicates that the selected calibrated equation $f_i(x)$ 116 is applied to the scaled waveform 110 to generate the calibrated pulse waveform 120. As mentioned, the selected calibration equation 116 produces a calibrated waveform 120 where its maximum and the minimum are estimates of invasively or non-invasively measured SP and DP, respectively, for the location at which the sensor 102 measures the non-invasive waveform. Block 122 indicates that the software detects the maximum and minimum values from the calibrated waveform 120 to estimate values for SP and DP. As mentioned, the purpose of the invention is for these values of SP and DP to closely estimate the invasively or non-invasively measured SP and DP.

The SP and DP values measured using the invention, can also be used to calibrate waveforms. For example, the current method can be used with a brachial cuff to capture an un-calibrated volumetric displacement waveform, and calibrate the waveform so that its minimum accurately estimates the patient's DP and its maximum accurately estimates the patient's SP. Without the calibration error, the transfer function method can be applied, if desired, to the calibrated brachial waveform to accurately determine the central aortic waveform without significant calibration error.

Figure 2 shows a system 200 configured in accordance with the second embodiment of the invention. Many aspects of system 200 shown in Fig. 2 are the same or similar to system 100 shown in Fig. 1. The same reference numbers are used in Fig. 2 for components that are the same as in Fig. 1. In general, the method of operation of system 200 in Fig. 2 is similar to the operation of system 100 in Fig. 1 through the processing step identified by block 112 in both Figures 1 and 2, when the respective systems 100, 200 detect parameter values for cardiovascular features in the scaled waveform 110. At this point in the process, the system 200 shown in Figure 2 deviates from the system 100 shown in Figure 1. In Figure 2, the detected features from block 112 are input for a classification algorithm 214 that determines a clinical classification 216 for which the patient's SP and DP are expected to qualify, such as optimal, normal, high normal, grade I hypertension and grade II hypertension based on American Heart association and European Society of Hypertension classification. (Chobanian A. et al "Seventh Report of the Joint National Committee on Prevention, Detection, Evaluation, and Treatment of High Blood Pressure" Hypertension 2003;42:1206-1252, and Mancia G et al "The task force for the management of arterial hypertension of the European Society of Hypertension" European Heart Journal 2007;28: 1462-1536) The classification algorithm 214 is described in more detail described below with respect to Figure 7.

The calibration equations 118 in the embodiment shown in Fig. 1 and the classification algorithm 214 shown in Fig. 2 can be determined by comparing non-invasively, un-calibrated collected data to invasively or non-invasively measured arterial pressure data. Data of un-calibrated, non-invasive peripheral or central arterial waveforms have been collected alongside recordings of invasively or non-invasively measured SP and DP values on a group representative of the general population (in term of age, height, weight, gender). Non-invasive un-calibrated arterial waveforms and invasively or non-invasively measured pressure values can be compared for measurements taken at the radial, finger, brachial and carotid arteries, respectively. The data in each case can be used to establish calibration equations (block 118) suitable to calculate SP and DP from the scaled pulse waveform 110.

Referring to Figure 3, a method of system identification can be used to establish the coefficients for proposed calibration equations 302. In this exemplary embodiment, the form of the calibration equations is a non-linear sigmoid function, which constitutes linear and nonlinear components. In general, the non-invasively un-calibrated collected waveform data is filtered and scaled, where Mx and Mn correspond to the maximum and minimum of the scaled non-invasive waveform. The scaled non-invasive un-calibrated waveform data is the input 300 for the proposed calibration equations 302. The calibrated waveform 304 for the respective artery, with its maximum and minimum values equal to (invasively or non-invasively) measured SP and DP, respectively, is the output of the proposed calibration equations 302. Given the known input 300 and output 304 from the collected data, calibration equations 302 with unknown coefficients are proposed. Then, the coefficients are estimated such that the difference between the equation output and the data collected for the blood pressure measurements is minimized. The calibration equations can theoretically be linear or non-linear or combination of both types, however, it has been found that using a non-linear component produces more accurate results.

In this example, the form of the proposed calibration equations 302, has linear and non-linear parts and can be expressed as follow:

$$y(t) = (X \times P_i) + \left( a_i \times f(X \times B_i + C_i) \right) + d_i \qquad [1]$$

where

$y(t)$ is the output waveform at time $t$

$P_i$, $B_i$, $C_i$ are matrices of coefficients for each equation $i$, and
$a_i$, $d_i$ are scalars (constants).

Vector X in equation [1] is a vector of delayed input and output values which can be represented as follow:

$$X = \begin{bmatrix} u(t) & u(t-1) \cdots & u(t-na)\, y(t-1) & \cdots & y(t-nb) \end{bmatrix} \qquad [2]$$

Where

$u(t)$ is the input waveform at time t,
$u(t-1)$ is the input waveform at time t-1,
$u(t-na)$ is the input waveform at time t-na,
$y(t-1)$ is the output waveform at time t-1,
$y(t-nb)$ is the input waveform at time t-nb, and
na, nb are the number of delay points for the input and output signals respectively.

In equation [1], $f()$ is a non-linear function which in this example is a sigmoid function expressed as follow:

$$f(z) = \frac{1}{e^{-z}+1} \qquad [3]$$

To illustrate how the equation work, let's assume that na and nb are equal to 1 then vector X in equation [1] will be

$$X = \begin{bmatrix} u(t) & u(t-1) & y(t-1) \end{bmatrix} \qquad [4]$$

Accordingly

$$P_i = \begin{bmatrix} p_1 \\ p_2 \\ p_3 \end{bmatrix} \qquad [5]$$

$$B_i = \begin{bmatrix} b_{1,1} & b_{1,2} & b_{1,3} \\ b_{2,1} & b_{2,2} & b_{2,3} \\ b_{3,1} & b_{3,2} & b_{3,3} \end{bmatrix} \qquad [6]$$

$$C_i = \begin{bmatrix} c_1 & c_2 & c_3 \end{bmatrix} \qquad [7]$$

Then substituting equations [4] to [7] into equation [1], the result will be

$$y(t) = \left( \begin{bmatrix} u(t) & u(t-1) & y(t-1) \end{bmatrix} \times \begin{bmatrix} p_1 \\ p_2 \\ p_3 \end{bmatrix} \right)$$
$$+ \left( a_i \times f \left( \begin{bmatrix} u(t) & u(t-1) & y(t-1) \end{bmatrix} \times \begin{bmatrix} b_{1,1} & b_{1,2} & b_{1,3} \\ b_{2,1} & b_{2,2} & b_{2,3} \\ b_{3,1} & b_{3,2} & b_{3,3} \end{bmatrix} + \begin{bmatrix} c_1 & c_2 & c_3 \end{bmatrix} \right) \right)$$
$$+ d_i$$

$$[8]$$

The aim of the system identification is to estimate coefficient matrices $P_i$, $B_i$, $C_i$ and the constants $a_i$, $d_i$ by minimizing the difference between estimated output 304 and the (invasively or non-invasively) measured pressure data.
Applying the system identification method on the (invasively or non-invasively) measured pressure data collected from a sample of the general population may for example result in five (5) different calibration equations $f_i(x)$

116 (see, Fig. 1) that can be implemented on the general population. In other words, the final form of the proposed calibration equations 302 in Figure 3 corresponds to the calibration equations $f_i(x)$ 116 programmed in to the system 100, and used in practice to detect peripheral or central SP and DP, depending on whether the system is designed to detect a peripheral waveform or a central waveform. The final form of the proposed calibration equations 302 is determined for different groupings of input 300 and output 304 waveform data, in which the groupings are based on waveform feature parameters determined by applying the system identification method. In the embodiment shown in Figure 1, the selection algorithm 114 can be, e.g., a decision tree that determines which calibration equation $f_i(x)$ 116 should be used based on waveform features.

Even though the input waveforms are scaled versions of un-calibrated, non-invasive waveforms, the method of determining the calibration equations results in the ability of the calibration equations $f_i(x)$ to shift the waveform and scale the amplitude of the waveform so that its minimum correlates with data collected for the patient's (invasively or non-invasively) measured DP and its maximum correlates with data collected for the patient's (invasively or non-invasively) measured SP. In other words, using machine learning or deep learning techniques, accurate information about measured SP and DP are extracted from the shape of the patient's un-calibrated, scaled non-invasive waveform.

Figure 4 describes cardiovascular related features of the scaled peripheral pulse waveform 110. Some of these features have been described e.g., in U.S. Patent No 5,265,011. Values for parameters pertaining to the features are used as inputs to the selection algorithm 114 for the embodiment shown in Figure 1 and for the classification algorithm 214 for the embodiment shown in Figure 2, in the case that the non-invasive waveform is a peripheral pulse waveform as distinguished from a central or carotid waveform. These features can be detected through the derivative method (as mentioned in U.S. Patent No 5,265,011) or any other suitable mathematical method in time or frequency like wavelet analysis. Exemplary features that can be used by the selection algorithm 114 or classification algorithm 214 include, for example, AIx, AUCs/AUCd, P1, P2, T1, T2, and ED as described in Figure 4. Other features like heart rate, cardiac period and slope of the systolic upstroke, which also can be detected from the scaled peripheral waveform, can also be used as input to the algorithms.

Figure 5 describes cardiovascular related features of a scaled central (e.g., carotid) pressure waveform, some of which were described in U.S. Patent No 5,265,011. Values for parameters pertaining to the features are used as inputs to the selection algorithm 114 for the embodiment shown in Figure 1 and for the classification algorithm 214 for the embodiment shown in Figure 2, in the case that the non-invasive waveform is a central or carotid pulse waveform as distinguished from a peripheral pulse waveform. These features can be detected through the derivative method (as mentioned in U.S. Patent No 5,265,011) or any other suitable mathematical method in time or frequency like wavelet analysis. Exemplary features that can be used by the selection algorithm 114 or classification algorithm 214 include, for example, AIx, AUCs/AUCd, T1, T2, and ED as described in Figure 4. Other features like heart rate, cardiac period and slope of the systolic upstroke, which also can be detected from the scaled central waveform, can also be used as input to the algorithms.

The selection algorithm 114, which selects the appropriate equation to estimate SP and DP from an un-calibrated arterial waveform based on the cardiovascular related features of the scaled waveform, can be developed using different machine learning methods like decision tree, support vector machine, linear and nonlinear regression, and neural network. For the resulting algorithm 114, the waveform's features are the input while the calibration equations 116 to estimate SP and DP from the scaled, un-calibrated arterial waveform are the output. As mentioned above, this is possible because known data representing the general population that includes waveform features are used to develop to calibration equations 116 and the selection algorithm 114.

Figure 6 illustrates one exemplary selection algorithm 114 in the form of a decision tree that is used to select a suitable calibration equation 116 based on the detected or calculated waveform features or parameters. The calibration equations 116 are labelled Eq1, Eq2, Eq3, Eq4 and Eq5 in Figure 6. The selected calibration equation (Eq1, Eq2, Eq3, Eq4 or Eq5) is used to estimate SP and DP from scaled, un-calibrated arterial waveform based on parameter values pertaining to the waveform features. In Figure 6, block 112 indicates that pulse waveform features 113 are detected or calculated from a scaled version of the non-invasively un-calibrated recorded pulse waveform 110. As mentioned, suitable feature detection methods, block 112, include the derivative method or other mathematical methods in time or frequency domain. The values detected or calculated pertaining to the waveform features 113 are the input to the decision tree 114, which in this example serves as the selection algorithm 114 in Figure 1. The decision tree 114 decides which calibration equation Eq1, Eq2, Eq3, Eq4 or Eq5 to use according to the values of the detected or calculated waveform features. Specifically, in Figure 6, one of five calibration equations (Eq1, Eq2, Eq3, Eq4 or Eq5) is selected based on values of AIx, ED, heart rate (HR) and the percentage ratio of AUCd to AUCs. The threshold values identified in Figure 6 are illustrative and are estimated based on data analysis, although additional data collection and analysis may result in modified values. Other examples may use more waveform features with more branches in the decision tree. Also, other algorithms that correlate the waveform features with the appropriate calibration equation like support vector machine, linear and nonlinear regression, and neural network can also be used as the selection algorithm.

Figure 7 pertains to the embodiment shown in Figure 2, where a classification algorithm 214 is used in place of a selection algorithm 114 and calibration equations 216 as in the embodiment in Figure 1. The classification algorithm 214 is developed to detect the hypertension (SP/DP) class (as classified by American Heart Association and European Society of Hypertension) from the scaled, un-calibrated arterial waveform 110 based on the recorded waveform cardiovascular related features. The classification algorithm 214 uses a machine learning method like decision tree, support vector machine, linear and nonlinear regression, and neural network. The waveform features are the input while the SP/DP class is the output. As mentioned above, this is possible because known data representing the general population that includes waveform features are used to develop to develop to the correlation with SP/DP classification.

Figure 7 illustrates one exemplary classification algorithm 114 in the form of a decision tree that is used to select a suitable SP/DP classification based on parameter values detected or calculated for cardiovascular waveform features in a peripheral or central waveform. The threshold values identified in Figure 7 are illustrative and are estimated based on data analysis, although additional data collection and analysis may result in modified values. In Figure 7, the SP/DP classifications are: Optimal [SP/DP<120/80 mmHg], Normal [120/80≤SP/DP<130/85], High Normal [130/85≤SP/DP<140/90], Grade I Hypertension [140/90≤SP/DP<160/100] and Grade I/II hypertension [160/100≤SP/DP]. Block 212 in Figure 7 indicates that pulse waveform features are detected from the non-invasively un-calibrated recorded, scaled waveform using detection methods like the derivative method or other suitable mathematical methods in time or frequency domain. The values for the detected waveform features 113 are the input to the decision tree 214 which according to the values of the identified waveform features selects the SP/DP class for the patient. In this example, the selection is based on values of AIx, ED, Heart rate (HR) and the percentage ratio of AUCd to AUCd. The value of the percentage ratio of AUCd to AUCd is used in the first step to determine whether the patient should be classified as having hypertension. If so the value of the augmentation index AIx is used to determine whether the hypertension is grade I or grade II. If the patient should not be classified as having hypertension, then the value of the time of the first systolic peak determines whether the patient should be classified as high normal versus normal or optimal. If the patient should be classified as normal or optimal, the value of ejection duration ED determines whether the patient should be classified normal or optimal. Other examples may use more waveform features with more branches of the tree decision. Other algorithm that correlates the waveform features with SP/DP class like support vector machine, linear and nonlinear regression, and neural network can also be used.

As mentioned, the decision trees in Figures 6 and 7 are meant to be illustrative. Moreover, it is expected that the structure of the decision tree my need to be more complicated than that shown in Figures 6 and 7 for the systems to accurately estimate invasive SP and DP, or hypertension classification, respectively.

## Claims

1.  A method of non-invasively measuring a patient's systolic and diastolic blood pressure comprising the steps of:

    providing a non-invasive sensor (102) to sense a raw signal representing un-calibrated arterial pulse waveform data for a patient;

    providing multiple calibration equations (116, 302), to a computing device, said multiple calibration equations to be applied to input data representing a scaled and filtered arterial pulse waveform (110) to generate output data representing a calibrated arterial pulse waveform (120), said multiple calibration equations being established based on arterial pulse waveforms and pressure data measured invasively or non-invasively from a sampling of the general population, wherein each calibration equation is determined for values of one or more parameters pertaining to cardiovascular features of the scaled and filtered arterial pulse waveform;

    non-invasively sensing, filtering and recording an un-calibrated arterial pulse waveform, wherein a signal from a sensor representing the un-calibrated arterial pulse waveform is filtered through a high pass filter and a low pass filter or through a band pass filter to provide a filtered, un-calibrated high-fidelity arterial pulse waveform (106) in which cardiovascular features of the waveform are preserved;

    said method further comprising the following steps performed using said computing device:

    scaling the recorded, un-calibrated pulse waveform such that the amplitude of the scaled waveform is set to a fixed value;

    determining values of one or more parameters pertaining to cardiovascular features of the scaled and filtered arterial pulse waveform (110);

    selecting one of the multiple calibration equations based on the determined values of the one or more parameters pertaining to cardiovascular features determined from the scaled and filtered arterial pulse waveform;

    applying the selected calibration equation (116, 302) to the scaled and filtered arterial pulse waveform (110) and outputting data representing a calibrated arterial pulse waveform (120) in which a maximum value of the calibrated arterial pulse waveform correlates with the systolic pressure for the patient and a minimum value of the calibrated arterial pulse waveform correlates with the diastolic pressure for the patient; and

estimating the patient's systolic peripheral blood pressure as the maximum value of the calibrated waveform and estimating the patient's peripheral diastolic blood pressure as the minimum value of the calibrated waveform.

2. The method as recited in claim 1 wherein the determined one or more cardiovascular parameters include augmentation index, ejection duration, and the ratio of area under the curve during diastole divided by the area under the curve during systole.

3. The method as recited in claim 1wherein the calibration equation is selected using a decision tree.

4. The method as recited in claim 1 wherein the multiple calibration equations are determined by comparing data collected for a sampling of the general population and comparing scaled, filtered, un-calibrated, non-invasive waveform data using a non-invasive sensor of the type used to record the raw signal from the patient to invasively measured waveform data collected from the respective subject in the general population..

5. The method as recited in claim 1 wherein the multiple calibration equations include linear components and non-linear components.

6. The method as recited in claim 5 wherein each of the multiple calibration equations as the following form:

$$y(t) = ([u(t) \quad u(t-1) \quad ... \quad u(t-na) \quad y(t-1) \quad \cdots \quad y(t-nb)] \times P_i)$$
$$+ \big( a_i$$
$$\times f([u(t) \quad u(t-1) \quad ... \quad u(t-na) \quad y(t-1) \quad \cdots \quad y(t-nb)] \times B_i + C_i) \big)$$

where

$y(t)$ is the output waveform at time t
$P_i$, is na+nb+1by 1 matrix of coefficients for recalibration equation $i$
$B_i$, is na+nb+1by na+nb+1 square matrix of coefficients for recalibration equation $i$
$C_i$ is na+nb+1by 1 matrix of coefficients for recalibration equation $i$
na, nb are the number of delay points for the input and output signals respectively,
$a_i$, $d_i$ are scalars (constants) for recalibration equation $i$
$u(t)$ is the input waveform at time t,
$u(t - 1)$ is the input waveform at time t-1,
$u(t - na)$ is the input waveform at time t-na,
$y(t - 1)$ is the output waveform at time t-1,
$y(t - nb)$ is the input waveform at time t-nb, and
and $f()$ is a non-linear sigmoid function expressed as follows:

$$f(z) = \frac{1}{e^{-z}+1} \quad .$$

7. The method as recited in any of the preceding claims wherein the un-calibrated pulse waveform that is non-invasively sensed and recorded is a peripheral waveform.

8. The method as recited in any of the preceding claims wherein the un-calibrated pulse waveform that is non-invasively sensed and recorded is a brachial cuff volumetric displacement waveform.

9. The method as recited in any of the preceding claims wherein the un-calibrated pulse waveform that is non-invasively sensed and recorded is a carotid waveform.

10. The method as recited in any of the preceding claims wherein said high pass filter cutoff frequency is in the range of 0.7 to 1 Hz and said low pass cutoff frequency is in the range of 30 to 40 Hz.

**Patentansprüche**

1. Verfahren zum nichtinvasiven Messen eines systolischen und diastolischen Blutdrucks eines Patienten, umfassend die folgenden Schritte:

   Bereitstellen eines nichtinvasiven Sensors (102) zum Erfassen eines Rohsignals, das nichtkalibrierte arterielle Pulswellenformdaten für einen Patienten darstellt;
   Bereitstellen mehrerer Kalibrierungsgleichungen (116, 302) an eine Rechenvorrichtung, wobei die mehreren Kalibrierungsgleichungen auf Eingabedaten anzuwenden sind, die eine skalierte und gefilterte arterielle Pulswellenform (110) darstellen, um Ausgabedaten zu generieren, die eine kalibrierte arterielle Pulswellenform (120) darstellen, wobei die mehreren Kalibrierungsgleichungen basierend auf arteriellen Pulswellenformen und Druckdaten, die invasiv oder nichtinvasiv gemessen wurden, aus einer Stichprobenprüfung der Allgemeinbevölkerung aufgestellt sind, wobei jede Kalibrierungsgleichung für Werte von einem oder mehreren Parametern bestimmt wird, die kardiovaskuläre Merkmale der skalierten und gefilterten arteriellen Pulswellenform betreffen;
   nichtinvasives Erfassen, Filtern und Aufzeichnen einer nichtkalibrierten arteriellen Pulswellenform, wobei ein Signal von einem Sensor, das die nichtkalibrierte arterielle Pulswellenform darstellt, durch einen Hochpassfilter und einen Tiefpassfilter oder durch einen Bandpassfilter gefiltert wird, um eine gefilterte, nichtkalibrierte arterielle Pulswellenform (106) hoher Genauigkeit bereitzustellen, in der kardiovaskuläre Merkmale der Wellenform erhalten bleiben;
   wobei das Verfahren ferner die folgenden Schritte umfasst, die unter Verwendung der Rechenvorrichtung durchgeführt werden:

   Skalieren der aufgezeichneten, nichtkalibrierten Pulswellenform, sodass die Amplitude der skalierten Wellenform auf einen festen Wert eingestellt ist;
   Bestimmen von Werten eines oder mehrerer Parameter, die kardiovaskuläre Merkmale der skalierten und gefilterten arteriellen Pulswellenform (110) betreffen;
   Auswählen einer der mehreren Kalibrierungsgleichungen basierend auf den bestimmten Werte des einen oder der mehreren Parameter, die kardiovaskuläre Merkmale betreffen, die aus der skalierten und gefilterten arteriellen Pulswellenform bestimmt werden;
   Anwenden der ausgewählten Kalibrierungsgleichung (116, 302) auf die skalierte und gefilterte arterielle Pulswellenform (110) und Ausgeben von Daten, die eine kalibrierte arterielle Pulswellenform (120) darstellen, wobei ein Maximalwert der kalibrierten arteriellen Pulswellenform mit dem systolischen Druck für den Patienten korreliert und ein Minimalwert der kalibrierten arteriellen Pulswellenform mit dem diastolischen Druck für den Patienten korreliert; und
   Schätzen des systolischen peripheren Blutdrucks des Patienten als den Maximalwert der kalibrierten Wellenform und Schätzen des peripheren diastolischen Blutdrucks des Patienten als den Minimalwert der kalibrierten Wellenform.

2. Verfahren nach Anspruch 1, wobei der bestimmte eine oder die bestimmten mehreren kardiovaskulären Parameter einen Augmentationsindex, eine Auswurfdauer und das Verhältnis der Fläche unter der Kurve während der Diastole geteilt durch die Fläche unter der Kurve während der Systole enthalten.

3. Verfahren nach Anspruch 1, wobei die Kalibrierungsgleichung unter Verwendung eines Entscheidungsbaums ausgewählt wird.

4. Verfahren nach Anspruch 1, wobei die mehreren Kalibrierungsgleichungen durch Vergleichen von Daten, die für eine Stichprobenprüfung der Allgemeinbevölkerung gesammelt wurden, und durch Vergleichen von skalierten, gefilterten, nichtkalibrierten, nichtinvasiven Wellenformdaten unter Verwendung eines nichtinvasiven Sensors des Typs, der verwendet wird, um das Rohsignal von dem Patienten aufzuzeichnen, mit invasiv gemessenen Wellenformdaten, die von dem jeweiligen Subjekt in der Allgemeinbevölkerung gesammelt wurden, bestimmt werden.

5. Verfahren nach Anspruch 1, wobei die mehreren Kalibrierungsgleichungen lineare Komponenten und nichtlineare Komponenten enthalten.

6. Verfahren nach Anspruch 5, wobei jede der mehreren Kalibrierungsgleichungen die folgende Form aufweist:

$$y(t) = ([u(t) \quad u(t-1) \quad \ldots \quad u(t-na) \quad y(t-1) \quad \cdots \quad y(t-nb)] \times P_i)$$
$$+ (a_i$$
$$\times f([u(t) \quad u(t-1) \quad \ldots \quad u(t-na) \quad y(t-1) \quad \cdots \quad y(t-nb)] \times B_i + C_i)) ,$$

wobei

> $y(t)$ die Ausgangswellenform zum Zeitpunkt $t$ ist,
> $P_i$ eine na+nb+1 x 1-Matrix von Koeffizienten für Rekalibrierungsgleichung $i$ ist,
> $B_i$ eine na+nb+1 x na+nb+1-Quadratmatrix von Koeffizienten für Rekalibrierungsgleichung $i$ ist,
> $C_i$ eine na+nb+1 x 1-Matrix von Koeffizienten für Rekalibrierungsgleichung $i$ ist,
> na, nb die Anzahl von Verzögerungspunkten für das Eingangs- beziehungsweise Ausgangssignal sind,
> $a_i$, $d_i$ Skalare (Konstanten) für Rekalibrierungsgleichung $i$ sind,
> $u(t)$ die Eingangswellenform zum Zeitpunkt t ist,
> $u(t-1)$ die Eingangswellenform zum Zeitpunkt t-1 ist,
> $u(t-na)$ die Eingangswellenform zum Zeitpunkt t-na ist,
> $y(t-1)$ die Ausgangswellenform zum Zeitpunkt t-1 ist,
> $y(t-nb)$ die Eingangswellenform zum Zeitpunkt t-nb ist und
> $f()$ eine nichtlineare Sigmoidfunktion ist, die wie folgt ausgedrückt wird:

$$f(z) = \frac{1}{e^{-z}+1} .$$

7. Verfahren nach einem der vorhergehenden Ansprüche, wobei die nichtkalibrierte Pulswellenform, die nichtinvasiv erfasst und aufgezeichnet wird, eine periphere Wellenform ist.

8. Verfahren nach einem der vorhergehenden Ansprüche, wobei die nichtkalibrierte Pulswellenform, die nichtinvasiv erfasst und aufgezeichnet wird, eine Volumenverschiebungswellenform der Oberarmmanschette ist.

9. Verfahren nach einem der vorhergehenden Ansprüche, wobei die nichtkalibrierte Pulswellenform, die nichtinvasiv erfasst und aufgezeichnet wird, eine Karotiswellenform ist.

10. Verfahren nach einem der vorhergehenden Ansprüche, wobei die Hochpassfilter-Grenzfrequenz in dem Bereich von 0,7 bis 1 Hz liegt und die Tiefpassfilter-Grenzfrequenz in dem Bereich von 30 bis 40 Hz liegt.

**Revendications**

1. Méthode de mesure non effractive de la tension artérielle systolique et diastolique d'un patient comprenant les étapes de :

> fourniture d'un capteur non effractif (102) pour détecter un signal brut représentant des données de forme d'onde de pouls artériel non étalonnée pour un patient ;
> fourniture de multiples équations d'étalonnage (116, 302), à un dispositif informatique, lesdites multiples équations d'étalonnage devant être appliquées à des données d'entrée représentant une forme d'onde de pouls artériel mise à l'échelle et filtrée (110) pour générer des données de sortie représentant une forme d'onde de pouls artériel étalonnée (120), lesdites multiples équations d'étalonnage étant établies sur la base de formes d'onde de pouls artériel et de données de pression mesurées de manière effractive ou non effractive à partir d'un échantillonnage de la population générale, chaque équation d'étalonnage étant déterminée pour des valeurs d'un ou plusieurs paramètres relatifs à des caractéristiques cardiovasculaires de la forme d'onde de pouls artériel mise à l'échelle et filtrée ;
> détection, filtrage et enregistrement non effractifs d'une forme d'onde de pouls artériel non étalonnée, un signal provenant d'un capteur représentant la forme d'onde de pouls artériel non étalonnée étant filtré à travers un filtre passe-haut et un filtre passe-bas ou à travers un filtre passe-bande de manière à fournir une forme d'onde de pouls artériel haute-fidélité filtrée non étalonnée (106) dans laquelle les caractéristiques cardiovasculaires de la forme d'onde sont conservées ;

ladite méthode comprenant en outre les étapes suivantes réalisées à l'aide dudit dispositif informatique :

la mise à l'échelle de la forme d'onde de pouls non étalonnée enregistrée, de sorte que l'amplitude de la forme d'onde mise à l'échelle soit réglée sur une valeur fixe ;

la détermination de valeurs d'un ou plusieurs paramètres relatifs à des caractéristiques cardiovasculaires de la forme d'onde de pouls artériel mise à l'échelle et filtrée (110) ;

la sélection de l'une des multiples équations d'étalonnage sur la base des valeurs déterminées desdits un ou plusieurs paramètres relatifs aux caractéristiques cardiovasculaires déterminées à partir de la forme d'onde de pouls artériel mise à l'échelle et filtrée ;

l'application de l'équation d'étalonnage sélectionnée (116, 302) à la forme d'onde de pouls artériel mise à l'échelle et filtrée (110) et la sortie de données représentant une forme d'onde de pouls artériel étalonnée (120) dans laquelle la valeur maximale de la forme d'onde de pouls artériel étalonnée est en corrélation avec la tension systolique pour le patient et la valeur minimale de la forme d'onde de pouls artériel étalonnée est en corrélation avec la tension diastolique pour le patient ; et

l'estimation de la tension artérielle périphérique systolique du patient en tant que valeur maximale de la forme d'onde étalonnée et l'estimation de la tension artérielle diastolique périphérique du patient en tant que valeur minimale de la forme d'onde étalonnée.

2. Méthode selon la revendication **1,** lesdits un ou plusieurs paramètres cardiovasculaires déterminés comprenant l'indice d'augmentation, la durée d'éjection et le rapport de l'aire sous la courbe pendant la diastole divisée par l'aire sous la courbe pendant la systole.

3. Méthode selon la revendication **1,** ladite équation d'étalonnage étant sélectionnée en utilisant un arbre de décision.

4. Méthode selon la revendication **1,** lesdites multiples équations d'étalonnage étant déterminées en comparant les données collectées pour un échantillonnage de la population générale et en comparant des données de forme d'onde non effractive, non étalonnées, filtrées et mises à l'échelle à l'aide d'un capteur non effractif du type utilisé pour enregistrer le signal brut du patient aux données de forme d'onde mesurées de manière effractive collectées auprès du sujet respectif dans la population générale.

5. Méthode selon la revendication **1,** lesdites multiples équations d'étalonnage comprenant des composantes linéaires et des composantes non linéaires.

6. Méthode selon la revendication 5, chacune des multiples équations d'étalonnage se présentant sous la forme suivante :

$$
\begin{aligned}
y(t) = &([u(t) \quad u(t-1) \quad ... \quad u(t-na) \quad y(t-1) \quad \cdots \quad y(t-nb)] \times P_i) \\
&+ (a_i \\
&\times f([u(t) \quad u(t-1) \quad ... \quad u(t-na) \quad y(t-1) \quad \cdots \quad y(t-nb)] \times B_i + C_i))
\end{aligned}
$$

où

$y(t)$ représente la forme d'onde de sortie à l'instant $t$,

$P_i$ représente la matrice de coefficients na+nb+ 1 par 1 pour l'équation de réétalonnage $i$,

$B_i$ représente la matrice carrée de coefficients na+nb+ 1 par na+nb+1 pour l'équation de réétalonnage $i$,

$C_i$ représente la matrice de coefficients na+nb+ 1 par 1 pour l'équation de réétalonnage $i$,

na, nb représentent le nombre de points de retard pour respectivement les signaux d'entrée et de sortie,

$a_i$, $d_i$ représentent des scalaires (constantes) pour l'équation de réétalonnage $i$,

$u(t)$ représente la forme d'onde d'entrée à l'instant $t$,

$u(t$ - 1) représente la forme d'onde d'entrée à l'instant $t$-1,

$u(t$ - $na)$ représente la forme d'onde d'entrée à l'instant $t$-na,

$y(t$ - 1) représente la forme d'onde de sortie à l'instant $t$-1,

$y(t$ - $nb)$ représente la forme d'onde d'entrée à l'instant $t$-nb, et

$f()$ représente une fonction sigmoïde non linéaire exprimée de la manière suivante :

$$f(z) = \frac{1}{e^{-z}+1}$$

7. Méthode selon l'une quelconque des revendications précédentes, ladite forme d'onde de pouls non étalonnée qui est détectée et enregistrée de manière non effractive étant une forme d'onde périphérique.

8. Méthode selon l'une quelconque des revendications précédentes, ladite forme d'onde de pouls non étalonnée qui est détectée et enregistrée de manière non effractive étant une forme d'onde de déplacement volumétrique de brassard brachial.

9. Méthode selon l'une quelconque des revendications précédentes, ladite forme d'onde de pouls non étalonnée qui est détectée et enregistrée de manière non effractive étant une forme d'onde de carotide.

10. Méthode selon l'une quelconque des revendications précédentes, ladite fréquence de coupure de filtre passe-haut étant comprise dans la plage de 0,7 à 1 Hz et ladite fréquence de coupure de filtre passe-bas étant comprise dans la plage de 30 à 40 Hz.

FIGURE 1

EP 3 609 393 B1

FIGURE 2

**Eq 1**

$$y(t) = (X \times P_1) + (a_1 \times f(X \times B_1 + C_1)) + d_1$$

**Eq 2**

$$y(t) = (X \times P_2) + (a_2 \times f(X \times B_2 + C_2)) + d_2$$

**Eq 3**

$$y(t) = (X \times P_3) + (a_3 \times f(X \times B_3 + C_3)) + d_3$$

Where
$u(t)$ is the input signal
$y(t)$ is the output signal
$X = [u(t) \; u(t-1) \cdots \; u(t-na) \; y(t-1) \; \cdots \; y(t-nb)]$
na, nb are the number of delay points for the input and output signals respectively
$P_i$, $B_i$, $C_i$ are matrix of coefficients for each model/equation i
$a_i$, $d_i$ are scalars (constants)

FIGURE 3

Features of Peripheral Pulse Waveform Scaled to Set Values Mx and Mn

$$AIx=(P1-P2)/(Mx-Mn)x\ 100\%$$

Mx=P1

P1-P2

P2

AUCs

Mn

AUCd

T1    T2    ED

Systole    Diastole

ED

T1: Time of the 1st systolic peak
T2: Time of the 2nd systolic peak
Mx: Pulse maximum
Mn: Pulse minimum
P1: 1st systolic peak
P2: 2nd systolic peak
ED: Ejection duration
AIx: Augmentation Index
AUCs = area under the curve during systole
AUCd = area under the curve during diastole

FIGURE 4

EP 3 609 393 B1

Features of Carotid Pressure Waveform Scaled to Set Values Mx and Mn

$$Alx = (P1-P2)/(Mx-Mn) \times 100\%$$

T1: Time of the 1st systolic peak
T2: Time of the 2nd systolic peak
Mx: Pulse maximum
Mn: Pulse minimum
P1: 1st systolic peak
P2: 2nd systolic peak
ED: Ejection duration
Alx: Augmentation index
AUCs = area under the curve during systole
AUCd = area under the curve during diastole

Mx=P1

P1-P2

P2

AUCs

Mn        T1        T2

AUCd

Systole        Diastole

ED

FIGURE 5

EP 3 609 393 B1

FIGURE 6

Scaled, Non-Invasive
Pulse Waveform → Waveform Features Detection ⟵ 212

Waveform Features

113

214

Example of a tree decision based on Waveform Features

(AUCd/AUCs)%≥120

No — AIx≥20 — Yes     Yes — T1≥100 — No

No — AIx≥20 — Yes

| Grade II/III Hypertension SP/DP≥160/100 | Grade I Hypertension 160/100>SP/DP≥140/90 |

No — ED≥300 — Yes

| Normal 130/85>SP/DP≥120/80 | Optimal SP/DP<120/80 |

| High Normal 140/90>SP/DP≥130/85 |

FIGURE 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8100835 B2
- US 20150327786 A1, Lading
- US 2012277602 A
- US 2016220194 A
- US 5265011 A
- US 9314170 B
- US 20110275944 A1

### Non-patent literature cited in the description

- **SHARMAN et al.** Validation of non-invasive central blood pressure devices: Artery Society task force consensus statement on protocol standardization. *European Heart Journal*, 2017, vol. 0, 1-10
- **CLOUD et al.** Estimation of central aortic pressure by SphygmoCor® requires intra-arterial peripheral. *Clinical Science*, 2003, vol. 105, 219-225
- **SHOJI et al.** Invasive validation of a novel brachial cuff-based oscillometric device (SphygmoCorXCEL) for measuring central blood pressure. *Journal of Hypertension*, 2016, vol. 34
- **MASÈ et al.** *Journal of Electrocardiology*, 2011, vol. 44, 201-207
- **CHEN et al.** *Annals of Biomedical Engineering*, 2012, vol. 40 (4), 871-882
- **ZHENG et al.** *J Med Syst*, 2016, vol. 40, 195
- **SOLÀ et al.** *35th Annual International Conference of the IEEE EMBS*, July 2013
- Photoplethysmography and ultrasonic-measurement-integrated simulation to clarify the relation between two-dimensional unsteady blood flow field and forward and backward waves in a carotid artery. **SONE SHUSAKU et al.** Medical And Biological Engineering And Computing. Springer, 27 July 2016, vol. 55, 719-731
- **W. NICHOLS** ; **M. O'ROURKE**. McDonald's Blood Flow in Arteries: Theoretical, Experimental and Clinical Principles
- **CHOBANIAN A. et al.** Seventh Report of the Joint National Committee on Prevention, Detection, Evaluation, and Treatment of High Blood Pressure. *Hypertension*, 2003, vol. 42, 1206-1252
- **MANCIA G et al.** The task force for the management of arterial hypertension of the European Society of Hypertension. *European Heart Journal*, 2007, vol. 28, 1462-1536